# EUROPEAN PATENT APPLICATION

(11) **EP 0 833 156 A1**
(43) Date of publication of application: **01.04.1998**
(21) Application number: 97109875.1
(22) Date of filing: 17.06.1997
(51) Int. Cl.: G01N 33/497, A61B 5/083, G01N 27/00

(54) **Method and device for detecting a variation in a flowing medium**

(30) Priority: 29.07.1996 SE 9602886
(71) Applicant: Siemens-Elema AB, 171 95 Solna 1 (SE)
(72) Inventor: Skog, Göran, 168 56 Bromma (SE)

(57) **Abstract**

In a method for detecting a variation, such as a variation in the concentration of a substance, in a flowing medium, whereby a sample of the medium is made to flow past a sensor (20, 24) for detecting the variation, the direction of flow of the sample is reversed and the velocity of the sample in relation to the sensor (20, 24) is reduced during the samples passage through the sensor. Thus, a device for detecting such a structure or variation contains a sensor (22, 24) and means (28, 32) for reversing the direction of flow and reducing the velocity of the sample of the medium.

## Description

The present invention relates to a method and device for detecting a variation, such as a variation in the concentration of a substance, in a flowing medium by passing a sample of said medium trough a sensor at a lower velocity for detecting the variation.

The long response times of gas sensors is a major disadvantage in many applications. Sensor sluggishness can lead to distorted results in e.g. the measurement of variation, such as rapid changes in the concentration of a substance in a flowing medium. Thus, a sluggish sensor would cause a steep pulse slope to be rendered with a lean slope. In the ventilator and anaesthesia arts, the slow response time is a particular problem with oxygen sensors but is also a problem with carbon dioxide sensors.

In avoiding the aforesaid problems with sluggish sensors, special measurement methods of varying complexity must often be used. See EP - B1 - 0 392 503 which describes a special technique for determining oxygen consumption and carbon dioxide output from breath to breath in a patient on a ventilator.

Another special measurement method is disclosed in US - A - 4,202,352, where exhaled gas from several breaths are stored in a gas storage tube. During analysis, the stored gas is passed through a gas analyser at a reduced velocity (compared with the velocity of the exhaled gas).

One drawback with this method, is the risk of diffusion of gas within the stored gas. In particular, the diffusion will be greatest within parts where rapid changes in concentration are present. There is also a risk that tube itself will cause contamination of the gas. This happens because gas particles near the wall of the tube can have other flow characteristics than gas particles in the middle of the tube (e. g. laminar and turbulent flows have different characteristics).

The objective of the present invention is to set forth a new technique for resolving the above problems in a simple fashion.

This objective is achieved with a method and a device of the initially described kind with the features set forth in claims 1 and 4 respectively.

When the sample is reversed in relation to the sensor the latest drawn part of the sample will first be analysed (with a lower velocity through the sensor). By controlling the sampling in an appropriate manner, the most interesting portions of the sample can hereby be analysed with no risk of diffusion or other contamination of the sample. If the sensor reproduces the variation in a generally accurate fashion, the exact variation can subsequently be electronically restored with the aid of an appropriate computer algorithm.

It should be noted that the above technique is suitable for use only if certain characteristics of the sample is of interest, since other information in the sample is lost.

According to advantageous embodiment of the invention, the sample of the medium is pumped from the flowing medium with a higher velocity than the velocity of the flowing medium. This contributes to enhance the resolution of rapid changes.

According to another advantageous embodiment of the device according to the invention, the sensor is placed to measure the variation as the pump draws the sample from the flowing medium. This naturally only provides for a coarse analysis. However, the coarse analysis can be used for spotting the interesting parts of the sample, and be used for controlling when to stop and reverse the flow with a lower velocity for detailed analysis of the interesting part.

According to yet another advantageous embodiment of the device according to the invention, several sensors are arranged to measure the variation of one substance at several locations or to measure the variation of several substances at one or several location(s).

The invention will now be explained in greater detail, referring to the attached drawing in which
FIG. 1 illustrates the application of the technique according to the invention to a ventilator system,
FIG. 2 shows a first suitable position for the sensor and pump with such a ventilator system,
FIG. 3 shows a second suitable position for the sensor and pump with such a ventilator system, and
FIG. 4 shows a diagram which schematically depict an advantageous effect of the method according to the invention.

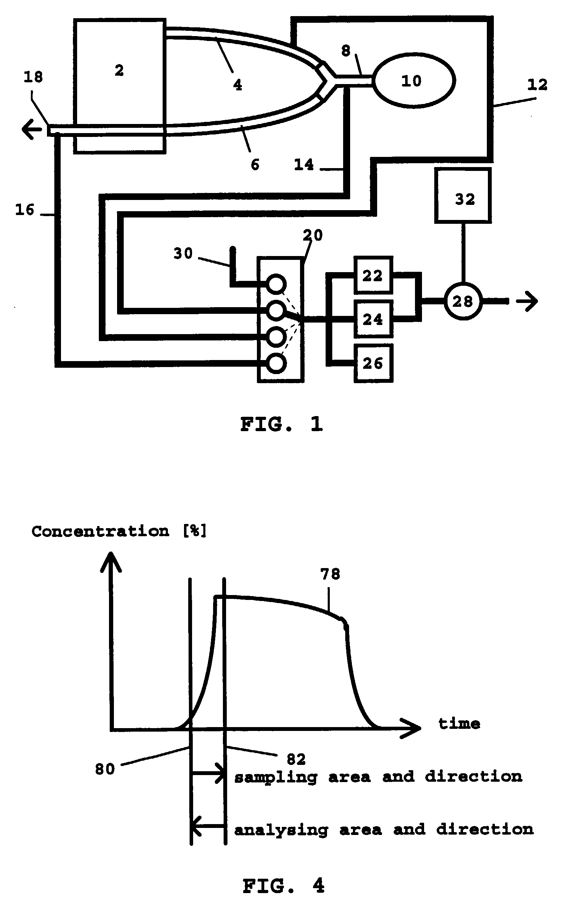

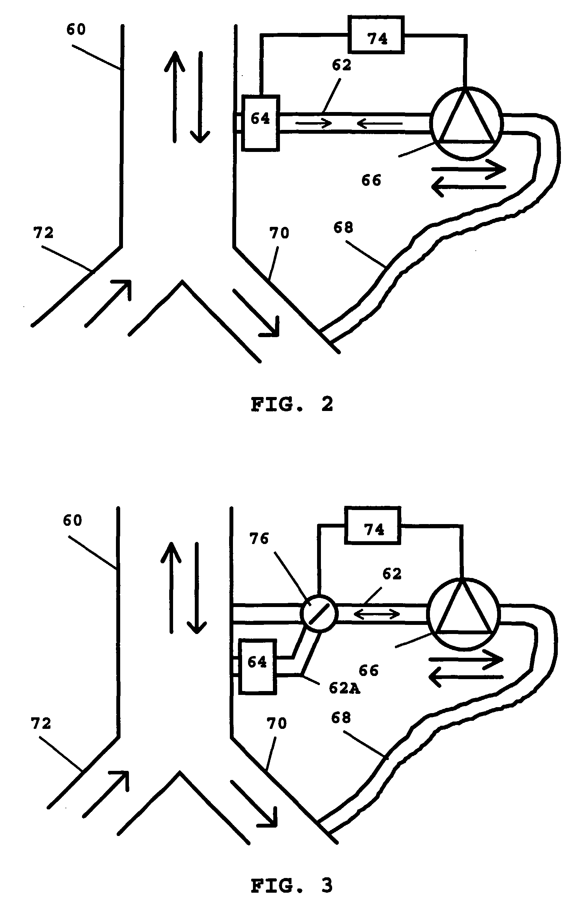

FIG. 1 shows a ventilator system with a ventilator 2, connected to a patient 10 via an inspiratory line 4, an expiratory line 6, and a Y piece 8. The patient is supplied with and relieved of a respiratory gas through the lines 4, 6, 8. There are also a first measurement tube 12 connected to the inspiratory line 4, a second measurement tube 14 connected to the Y piece 8 and, a third measurement tube 16 connected to the expiratory line 6. The third measurement tube 16 is connected close to the expiratory line's 6 outlet 18 to ambient air. The measurement tubes 12, 14, 16 can be selectively connected, via a multiplex valve 20, to sensors 22, 24 and pressure gauge 26 for analysing the respiratory gas. These sensors 22, 24 can consist of an oxygen sensor 22 and a carbon dioxide sensor 24. A pump 28 is arranged to pump specimens or samples of inspiratory and expiratory gases through the measurement tubes 12, 14, 16, via the multiplex valve 20, to the sensors 22, 24 and pressure gauge 26 for analysis. The sensors 22, 24 can also be connected, via an additional tube 30 connected to the multiplex valve 20, to ambient air or other suitable location (as disclosed below). Likewise, the outlet of the pump 28 can be connected to ambient air or other suitable location.

The pump's 28 pumping rate and pumping direction is controlled by a control means 32. In the embodiment of FIG. 1, the sensors 22, 24 and pressure gauge 26 are situated at a distance from respective measurement point (connection point with inspiratory line 4, Y piece 8 and expiratory line 6). In order to achieve relatively short analysis times of samples, the pump 28 is controlled to draw a sample from respective line at a fast rate (faster than the flow of the respiratory gas itself) for transporting samples from the sampling site on the inspiratory line 4, expiratory line 6 and Y piece 8 respectively to the sensors 22, 24 and pressure gauge 26. This also enhances the resolution of rapid changes in respiratory gas. Pressures measured by the pressure gauge are corrected for the influence of the measurement lines 12, 14, 16 in known manner. When a sample has been pumped past the sensors 22, 24 (and resides between the sensors 22, 24 and the pump 28) the pumping direction is reversed and the rate (velocity) is reduced to a slower pumping rate to pump the sample past the sensor 22, 24 in reversed direction.

This approach improves the ability of the sensors 22, 24 to measure rapid variations or changes in the samples without the loss of information. If e.g. the steep forward flank of a pulse is of interest, i.e. the pulse's rise time, and this pulse is pumped at "full" speed through a sensor, whose maximal rise time is much slower, the sensor will reproduce the forward flank with a slope greater than the true slope, and the measurement will accordingly be erroneous. As noted above, the problem of sensor sluggishness is greatest with oxygen sensors. However, if the velocity of the flank past the sensor is slowed, the problem of a sluggish sensor can be reduced, and the sample can subsequently be electronically restored, with the aid of a suitable algorithm, to its original conformation.

Since the sample passes the sensors 22, 24 when it passes towards the pump, the signal from the sensors 22, 24 can be used (albeit the sluggishness of the sensors 22, 24) to indicate when the interesting part of the sample has passed the sensors 22, 24. Upon such indication (e. g. a steep forward flank), the pump 28 is stopped and reverse its pumping at a slower speed for detailed analysis of the interesting part of the sample. This also means that the most interesting part of the sample to analyse, will enter the sensors 22, 24 first, thereby reducing diffusion and contamination of the sample. This will be even clearer in connection with FIGS. 2 and 3 below.

The multiplex valve 20 can remain in the same state (allowing the sample to be pumped back towards the sample site) or switched to connect with tube 30. If switched to connect with tube 30, the sample can be pumped into ambient air. However, the tube 30 can also be connected to an evacuation system for collecting all samples or even connected to another part of the respiration lines (inspiratory line 4, expiratory line 6, Y piece 8). The same can be said about the outlet of the pump 28. This can also be connected to an evacuation system for collecting gas or even connected to another part of the respiration lines (inspiratory line 4, expiratory line 6, Y piece 8).

So for the effect of the invention to be achieved, the direction of flow of the sample is reversed and the velocity of the sample reduced in relation to the velocity of the medium to be analysed (in this case respiratory gas).

In the system in FIG. 1, measurements of e.g. oxygen can be made in the inspiratory line 4, through the measurement tube 12, and in the expiratory line 6, through the measurement tube 16, in determinations of the patient's 10 oxygen consumption. The end tidal sample is taken from the Y piece 8, through the measurement tube 14 in the known manner, for determination of the carbon dioxide concentration.

FIG. 2 shows a Y piece similar to the one in the ventilator system in FIG. 1. In this embodiment, however, a different approach to placement of sensors is disclosed. A branch tube 62 is connected to the Y piece. A sensor 64 is arranged to analyse samples from the Y piece 60 in the branch tube 62. Samples can be extracted from the main flow of respiratory gas through the Y piece 60. With this location of the sensor 64, analysis can be made almost immediately after the sample has been taken. A pump 66 is arranged to pump a sample from the Y piece 60 and reverse the flow (as indicated by the arrows) to pump the sample, at a slower rate, through the sensor 64 (for analysis) back to the Y piece 60. If controlled accurately, the pump 66 could draw the precise volume to be analysed, and return this to the Y piece 60.

In the embodiment shown in FIG. 2, however, a return tube 68 from the pump 66 passes excess gas over to the expiratory side 70 (the inspiratory side being designated 72). (Similar to what was described in connection with the embodiment of FIG. 1, such excess, or surplus gas can be discarded completely to ambient air or to an evacuation system.)

The sensor 64 is connected to a control means 74 for the pump 66. As described above, a coarse analysis can be made as sample gas is drawn through the sensor 64 towards the pump 66 for identifying interesting parts of the sampled gas, whereupon the pump 66 is reversed and slowed down for detailed analysis.

One important advantage of this method is shown in the diagram in FIG. 4. The diagram shows concentration and time for a curve 78 (e. g. a CO₂-curve). The curve 78 includes two steep parts with rapid changes. A sampling area is indicated between two lines 80, 82. An upper arrow shows the time-based sample direction, i. e. from zero to maximum concentration. When analysing the sample with reversed flow, the time-based analysing direction will be the opposite as indicated with the lower arrow, i. e. the steep rise will be analysed from maximum towards minimum. Hereby, the most interesting part of the sample (near the maximum) will be analysed first, giving practically no time for diffusion of CO₂ to parts of the sample having lower concentration of CO₂.

FIG. 3 shows an alternative embodiment to the one disclosed in FIG. 2. Components and elements that can be identical have the same designation numbers. The major difference is that the branch tube 62 comprise a separate return tube 62A, in which the sensor 62 is placed. When a sample is drawn from the Y piece 60 through branch tube 62 it will pass a valve 76, which is switched to conduct the sample to the return tube 62A when the pump 66 is reversed.

## Claims

1. A method for detecting a variation, such as a variation in the concentration of a substance, in a flowing medium, a sample of the medium being made to flow past a sensor (22, 24; 64) with a velocity that is lower than the velocity of the medium for detecting the variation, **characterized in** that the flow direction of the sample is reversed before passing the sample through the sensor (22, 24; 64) at the lower velocity.

2. A method according to claim 1, **characterized in** that the sample is drawn from the flowing medium with a velocity that is higher than the velocity of the medium.

3. A method according to claim 1 or 2, **characterized in** that a coarse detection of the variation is made when the sample is drawn from the flowing medium for identifying a specific characteristic in the sample and when the specific characteristic is identified, the flow direction of the sample is reversed to pass the sensor (22, 24; 64) with reduced velocity for detailed detection of the specific characteristic.

4. A device, for detecting a variation, such as a variation in the concentration of a substance in a flowing medium, comprising a sensor (22, 24; 64) which a sample of the medium passes for detection of the variation and a pump (28; 66) for drawing the sample from the flowing medium and pumping it through the sensor (22, 24; 64) and a control means (32; 74) for controlling the pump (28; 66), **characterized in** that the control means (32; 74) controls the pump (28; 66) to draw a sample from the flowing medium and then reverse the direction of flow for the sample and pump the sample at a lower velocity through the sensor (22, 24; 64).

5. A device according to claim 4, **characterized in** that the sensor (64) is arranged at the point where the sample is drawn from the flowing medium, whereby the pump (66) is controlled to first draw the sample from the flowing medium through the sensor (64) and then reverse the direction of flow for the sample and pump the sample at the lower velocity through the sensor (64), back to the flowing medium.

6. A device according to claim 4 or 5, **characterized in** that the control means (32; 74) is connected to the sensor (22, 24; 64) and controls the reversal of the pump (28; 66) depending on a measurement signal from the sensor (22, 24; 64) when a sample is drawn.

7. A device according to any of the claims 4-6, **characterized in** that the control means (32; 74) controls the pump (28; 66) to draw a sample from the flowing medium with a velocity that is higher than the velocity of the flowing medium.

8. A device according to any of claims 4-7, **characterized in** that a plurality of sensors (22, 24) is arranged to measure a plurality of different variations in the flowing medium, such as the variations in concentration of a plurality of different substances.

9. A device according to any of claims 4-8, **characterized in** that the flowing medium is inspiratory gas from a ventilator (2) or anaesthetic machine to a patient (10).

10. A device according to any of claims 4-9, **characterized in** that the flowing medium is expiratory gas to a ventilator (2) or anaesthetic machine from a patient (10).
